# EUROPEAN PATENT SPECIFICATION

(11) **EP 3 117 212 B1**
(45) Date of publication and mention of the grant of the patent: **30.05.2018**
(21) Application number: 14824148.2
(22) Date of filing: 11.12.2014
(51) Int. Cl.: G01N 33/50

(54) **METHOD OF STUDYING ABCG2 PROTEIN ACTIVITY AND USE OF 2-AMINOETHYL DIPHENYLBORINATE**
VERFAHREN ZUM STUDIEREN DER ABCG2-PROTEIN-AKTIVITÄT UND VERWENDUNG VON 2-AMINOETHYL-DIPHENYLBORINAT
PROCÉDÉ D'ÉTUDE DE L'ACTIVITÉ DE LA PROTÉINE ABCG2 ET UTILISATION DE 2-AMINOÉTHYL DIPHENYLBORINATE

(30) Priority: 10.03.2014 PL 40745314
(43) Date of publication of application: 18.01.2017
(73) Proprietor: Uniwersytet Lodzki, 90-131 Lodz (PL)
(72) Inventor: STUDZIAN, Maciej, 91-336 Lodz (PL); PULASKI, Lukasz, PL 90-042 Lodz (PL)
(74) Representative: Witek, Rafal
(86) International application number: PCT/PL2014/050076
(87) International publication number: WO 2015/137832

(56) References cited:
- WO-A1-2013/128217
- H. HU ET AL: "Two amino acid residues determine 2-APB sensitivity of the ion channels TRPV3 and TRPV4", PROCEEDINGS OF THE NATIONAL ACADEMY OF SCIENCES, vol. 106, no. 5, 3 February 2009 (2009-02-03), pages 1626-1631, XP055172665, ISSN: 0027-8424, DOI: 10.1073/pnas.0812209106
- ROBERT W ROBEY ET AL: "ABCG2: determining its relevance in clinical drug resistance", CANCER AND METASTASIS REVIEWS, KLUWER ACADEMIC PUBLISHERS, DO, vol. 26, no. 1, 24 February 2007 (2007-02-24), pages 39-57, XP019478295, ISSN: 1573-7233, DOI: 10.1007/S10555-007-9042-6

## Description

The object of the invention is a method of determining the transport activity level of the ABCG2 protein, particularly in a studied mammalian cell, and use of 2-aminoethyl diphenylborinate to perform such a method. The method may be used in particular to search for new medicaments, particularly of a polyphenol compounds group.

Human multidrug resistance protein ABCG2 is one of membrane transporters. The protein transports many substances of pharmacological importance - metabolites, nutrients, medicaments, out of the cells. Therefore, the protein may also regulate the rate of penetration of therapeutic substances into the cell. For example, there often occurs a pathological overexpression thereof in cancer cells, whereby they become insensitive to chemotherapy. Inhibition of the activity of these proteins by use of inhibitors is one of the strategies in development of new anti-cancer drugs. Physiological presence of the ABCG2 protein in tissue barrier cells (e.g. blood-brain, blood-placenta, blood-testis barrier) may significantly change the distribution of drugs in the body, preventing a therapeutic substance from reaching its target site. Evaluation of the ABCG2 protein activity in the studied material or testing the influence of a potential substrate on the activity of this protein may be of great clinical importance, e.g. when selecting an effective chemotherapy method, or it may find practical use during development of new drugs or studying of drug interactions in vitro.

The known tests for the ABCG2 protein activity utilize fluorescent substrates (e.g.Hoechst dye, BODIPY-prazosin, pheophorbide A, etc.) which are available in small selection, have low fluorescence efficiency thereby decreasing the method sensitivity and additionally many of these are also substrates for other multidrug resistance proteins (especially ABCB1) lowering the specificity of the measurements and necessitating the use of specific inhibitors.

2-aminoethyl diphenylborinate (also abbreviated as APB, CAS no. 208-366-5) is used in mammalian biology as an inhibitor of intracellular calcium channels, while in phytochemistry it is sometimes used as a derivatization reagent for detection of polyphenols (particularly flavonoids) forming a colored and fluorescent adduct therewith in a non-enzymatic reaction. It is most commonly used for derivatization of compounds separated by thin-layer chromatography and for densitometric detection of flavonoids in such preparations. It is also sometimes employed in plant histochemistry for detection of flavonoids in plant tissues under a fluorescence microscope.

Despite continuous progress in development of methods for measuring the activity of multidrug resistance proteins there is still a need for improved techniques allowing for more accurate studies. It is particularly desired to obtain a method based on using fluorescence measurement that would be correlated with the activity of the ABCG2 protein or a derivative thereof.

It has been surprisingly found that the above mentioned problem is solved by the present invention.

The object of the invention is use and methods defined in detail in the appended claims.

### Detailed description of the invention

The invention relates to use of 2-aminoethyl diphenylborinate for studying and practically testing the activity of ABCG2 in order to:
a) identify new ABCG2 substrates among polyphenolic compounds;
b) characterize enzymatic/kinetic properties of ABCG2 variants (sequential variants, chemically modified variants, variants from different species);
c) test the level of ABCG2 activity in clinical samples: biopsies, cancer cells preparations etc.;
d) identify new ABCG2 inhibitors for clinical use by screening chemical libraries activities against flavonoid transport by ABCG2;
e) detect pharmacokinetic interactions with ABCG2 (ABCG2 inhibition and/or being transported by ABCG2) for compounds of pharmacological importance: medicaments used in clinical practice, food ingredients, para-pharmaceuticals, elements of environmental pollution etc.;
f) study the effect of physical factors (temperature, irradiation, osmotic pressure etc.) on ABCG2 activity.

APB is used in all of the cases above for quantitative derivatization of flavonoids (model ones, with the best kinetic parameters, in particular such as luteolin; or those required by the given task, in particular the studied compounds with unknown activity towards ABCG2) in biological compartments (cells, membrane vesicles, sub-cellular structures), wherefrom or whereto flavonoid transport mediated by ABCG2 occurs. In an exemplary embodiment, measurement is conducted by one of the quantitative fluorimetric detection techniques, namely with a fluorimeter, microplate reader, flow cytometer, imaging flow cytometer, fluorescence microscope, confocal microscope, automated fluorescence microscope / HCS (high content screening) device etc. APB, which easily and quickly crosses biological membranes, including mammalian ones, was used according to the present invention for quantitative measurement of flavonoid concentration in cells and cell-derived biological systems of mammalian origin. It was shown that derivatization occurs almost immediately and quantitatively, the resulting adduct fluorescence being proportional to flavonoid concentration in the starting sample and that it may be measured in a homogeneous system (in a cuvette spectrofluorimeter or a fluorimetric microplate reader). According to the invention, it was possible to demonstrate flavonoid transport by the ABCG2 protein directly in various biological systems containing ABCG2, such as: cells naturally containing the protein, transgenic cells where it had been introduced by means of genetic engineering, and membrane vesicles (cell-derived) containing the protein. In these systems it was possible to show that flavonoid transport is caused by ABCG2, by using specific inhibitors and by utilizing systems with varied (known) ABCG2 content. In particular, the transport by ABCG2 was measured fortheseflavonoids/polyphenols, which were known to be transported by this protein (quercetin, kaempferol). By using the invention, the transport of compounds which until now were described in literature only as ABCG2 inhibitors (chrysin, luteolin) was also shown, as well as for those for which their interactions with ABCG2 were not previously known. According to the invention, direct kinetic parameters were determined (transport rate, affinity) of the interactions of exemplary flavonoids with ABCG2.

### Example 1. Verification of the possibility of polyphenolic substrate transport by the ABCG2 protein

The set of 10 polyphenolic compounds was purchased at TimTec (www.timtec.net):
Compound 1: quercetin (CAS no. 117-39-5)
Compound 2: kaempferol (CAS no. 520-18-3)
Compound 3: luteolin (CAS no. 491-70-3)
Compound 4: fisetin (CAS no. 528-48-3)
Compound 5: 5,3',4'-trihydroxyflavone (CAS no. 19852-25-6)
Compound 6: 6,3',4'-trimethoxy-3-hydroxyflavone (no CAS number)
Compound 7: myricetin(CAS no. 529-44-2)
Compound 8: 3,5-dihydroxyflavone (CAS no. 6665-69-6)
Compound 9: 2',5'-dimethoxy-3-hydroxyflavone (no CAS number)
Compound 10: 3',4'-dimethoxy-3-hydroxy-6-methylflavone (CAS no. 93097-20-2)

Stock solutions of the polyphenolic compounds in dimethyl sulfoxide (DMSO) were prepared with concentrations of 10 mg/ml.

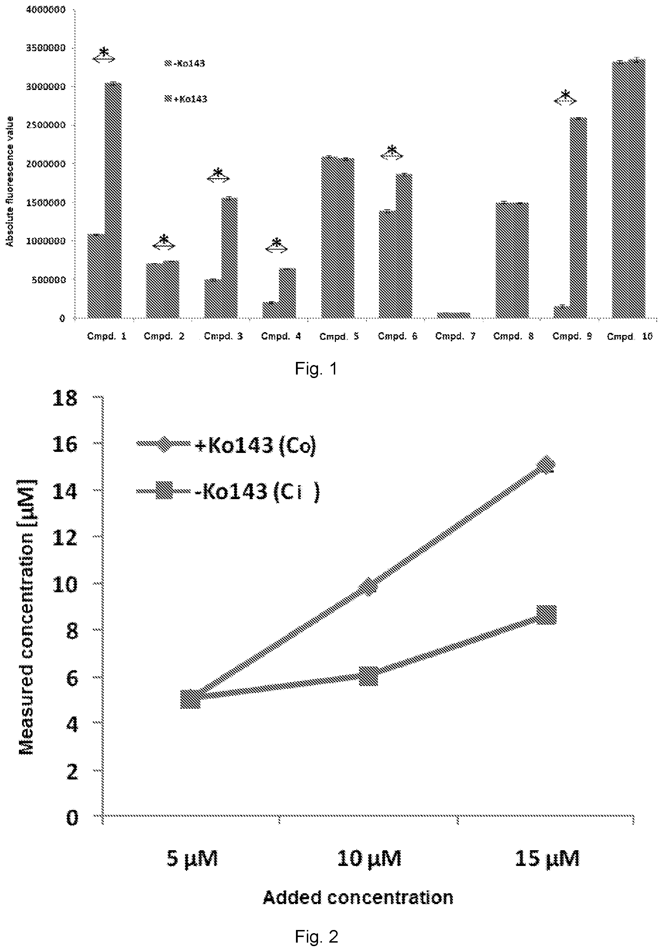

24 hours before the experiment, cells of the SB MDCKII-BCRP cell line (purchased from Solvo Biotechnology) in continuous in vitro culture, exhibiting stable overexpression of human ABCG2 protein, were plated on a standard black 96-well polystyrene microplate with surface modified for cell culture (product from Thermo Scientific Nunc cat. no. 137101) with 50,000 cells per well in standard DMEM culture medium (product from LifeTechnologies cat. no. 32430) supplemented with 10% fetal bovine serum. The experiment was started by removing the culture medium from the cells and replacing it with medium of the same composition (in volume of 100 µl) supplemented with the studied compound with a final concentration of 20 µM. Medium with each of the studied compounds was added to ten wells on the plate, the medium also containing 10 µM of compound Ko143 (known inhibitor of the ABCG2 protein, product from Sigma Aldrich, cat. no. K2144) in five of the wells - these wells were designated as '+Ko143', and five wells not containing this additive - these wells were designated as '-Ko143'. The cells were then incubated for 1 h in a standard incubator for in vitro cell culture in temperature of 37°C. The incubation was followed by removing the medium and the cells were washed twice with portions of 50 µl of buffered saline (PBS, product from Life Technologies cat. no. 10010) in temperature of 4°C. Then the cells were lysed (solubilized) in 100 µl of PBS with the following additives: 0.5% (v/v) Triton X-100 surfactant (product from Sigma Aldrich, cat. no. T8787) and 0.25 mg/ml 2-aminoethyl-diphenylborinate (APB, CAS no. 208-366-5, product from Sigma Aldrich, cat. no. D9754). The compound APB was added to the PBS solution from a stock solution in DMSO with a concentration of 250 mg/ml permanently stored in temperature of -20°C. The cells were lysed by shaking the microplate in room temperature for 5 minutes. This was followed by measurement of fluorescence (deriving from conjugates of the polyphenolic compounds with APB) using a fluorimetric microplate reader (device EnVision from Perkin Elmer) with excitation wavelength of 485 nm and emission wavelength of 535 nm, 555 nm or 579 nm (results with highest absolute values were chosen for interpretation). The results are shown on Fig. 1. Statistically significantly higher value of fluorescence in the presence of Ko143, a known inhibitor of substrate transport by the ABCG2 protein, suggests that when the ABCG2 protein activity is inhibited more of the given polyphenolic compound is accumulated inside the cells, and thus the given compound is a transport substrate for the ABCG2 protein. Based on the obtained results it can be inferred that the ABCG2 protein substrates are (among the studied ones) compounds 1, 2, 3, 4, 6 and 9. The relative difference between the fluorescence values leads to the conclusion that transport capacity is the highest for compounds 9, 3 and 1 and the lowest for compounds 2 and 6.

On Figure 1 fluorescence of the conjugates of APB and polyphenolic compounds is shown for the cell lysates (mean ± the standard error of the mean, n=5; statistically significant differences at the significance level <0.05 are marked with * symbol).

### Example 2. Testing of kinetic parameters for the polyphenolic compounds transport by the ABCG2 protein

The set of three polyphenolic compounds was purchased from Sigma Aldrich:
Quercetin - cat.np. Q0125
Luteolin - cat.no. L9283
Kaempferol - cat.no. 60010

Stock solutions of the polyphenolic compounds in dimethyl sulfoxide (DMSO) were prepared with concentrations of 50 mM.

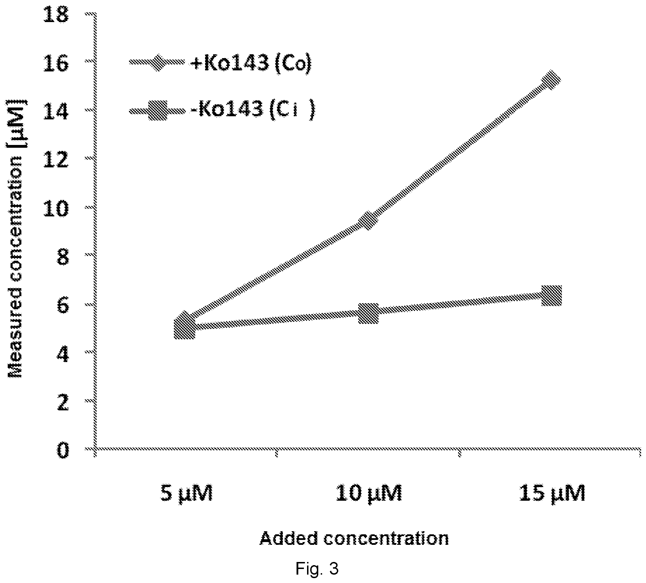

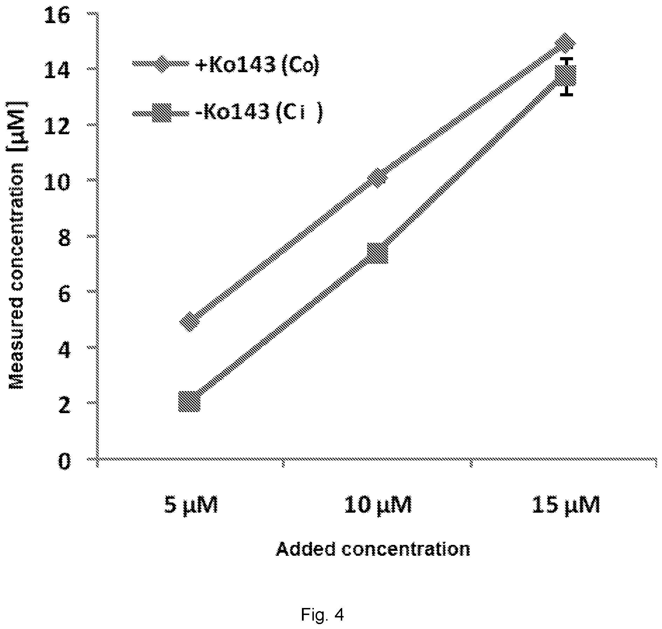

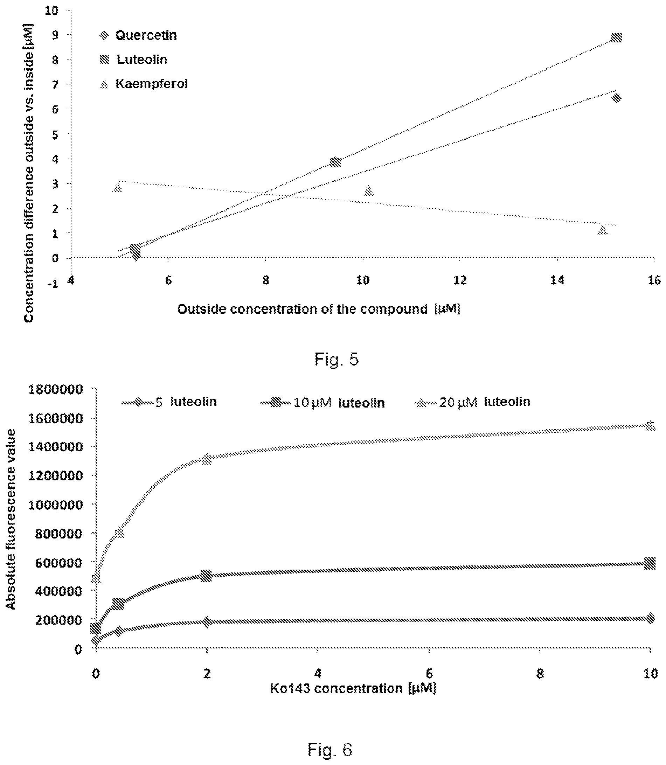

24 hours before the experiment, cells of the SB MDCKII-BCRP cell line (purchased from Solvo Biotechnology) in continuous in vitro culture, exhibiting stable overexpression of human ABCG2 protein, were plated on a standard black 96-well polystyrene microplate with surface modified for cell culture (product from Thermo Scientific Nunc cat. no. 137101) with 50,000 cells per well in standard DMEM culture medium (product from LifeTechnologies cat. no. 32430) supplemented with 10% fetal bovine serum. The experiment was started by removing the culture medium from the cells and replacing it with medium of the same composition (in volume of 100 µl) supplemented with the studied compound with a final concentration of 5, 10 or 20 µM. Medium with each of the concentrations of the studied compound was added to ten wells on the plate, the medium also containing 10 µM of compound Ko143 (known inhibitor of the ABCG2 protein, product from Sigma Aldrich, cat. no. K2144) in five of the wells - these wells were designated as '+Ko143', and five wells not containing this additive - these wells were designated as '-Ko143'. The cells were then incubated for 1 h in a standard incubator for in vitro cell culture in temperature of 37°C. The incubation was followed by removing the medium and the cells were washed twice with portions of 50 µl of buffered saline (PBS, product from Life Technologies cat. no. 10010) in temperature of 4°C. Then the cells were lysed (solubilized) in 100 µl of PBS with the following additives: 0.5% (v/v) Triton X-100 surfactant (product from Sigma Aldrich, cat. no. T8787) and 0.25 mg/ml 2-aminoethyl-diphenylborinate (APB, CAS no. 208-366-5, product from Sigma Aldrich, cat. no. D9754). The compound APB was added to the PBS solution from a stock solution in DMSO with a concentration of 250 mg/ml permanently stored in temperature of -20°C. The cells were lysed by shaking the microplate in room temperature for 5 minutes. This was followed by measurement of fluorescence (deriving from conjugates of the polyphenolic compounds with APB) using a fluorimetric microplate reader (device EnVision from Perkin Elmer) with excitation wavelength of 485 nm and emission wavelength of 535 nm for kaempferol, 555 nm for quercetin and 579 nm for luteolin. For the purpose of interpretation of the results, according to literature data it was assumed that in the '+Ko143' wells there was a complete inhibition of transport activity of the ABCG2 protein and equalization of concentrations inside and outside the cell and thus the fluorescence values measured for these wells were used as a calibration curve in a method of determining the intracellular concentration of the studied compound (the linear nature of the dependence of the measured fluorescence on the concentration of the studied compound confirmed this assumption), and dependence curves of the studied compounds concentrations in the cells in the presence and absence of the inhibitor on the extracellular concentration were plotted (Fig. 2-4). In order to determine the kinetic parameters (apparent maximum transport rate), while assuming that equilibrium has been established and the rates of transport into the cell (diffusion transport, driven by the difference in concentrations) and out of the cell (active transport by the ABCG2 protein) are equal, the dependence of the measured difference in concentrations outside and inside the cell on the external concentration of the studied compound was plotted for each of the studied compounds (Fig. 5). In the case of equilibrium nature of the process, the graph should be linear and its slope characterizing the ratio of maximum rate of active transport to the affinity of the transporter for the substrate (specificity constant), while taking the passive diffusion constant into account. Based on the obtained results it can be inferred that luteolin and quercetin are transported with a similar ratio of rate to affinity, while the low slope of the curve for kaempferol shows that it is being transported with low maximum rate and probably with high affinity, whereas the predominant role is played by a high passive diffusion constant.

Figures 2-4 show the measured (based on the fluorescence of the conjugate with APB) concentrations of the studied compounds (Fig. 2 - quercetin, Fig. 3 - luteolin,

Fig. 4 - kaempferol) in the cells, in the presence and absence of the ABCG2 protein inhibitor (corresponding to Co - concentration outside the cell and Ci - concentration inside the cell at the steady state). Mean ± the standard error of the mean, n=5.

### Example 3. Testing of inhibition parameters for the transport activity of the ABCG2 protein

Luteolin (cat. no. L9283) was purchased from Sigma Aldrich and a stock solution thereof in dimethyl sulfoxide (DMSO) was prepared with a concentration of 50 mM.

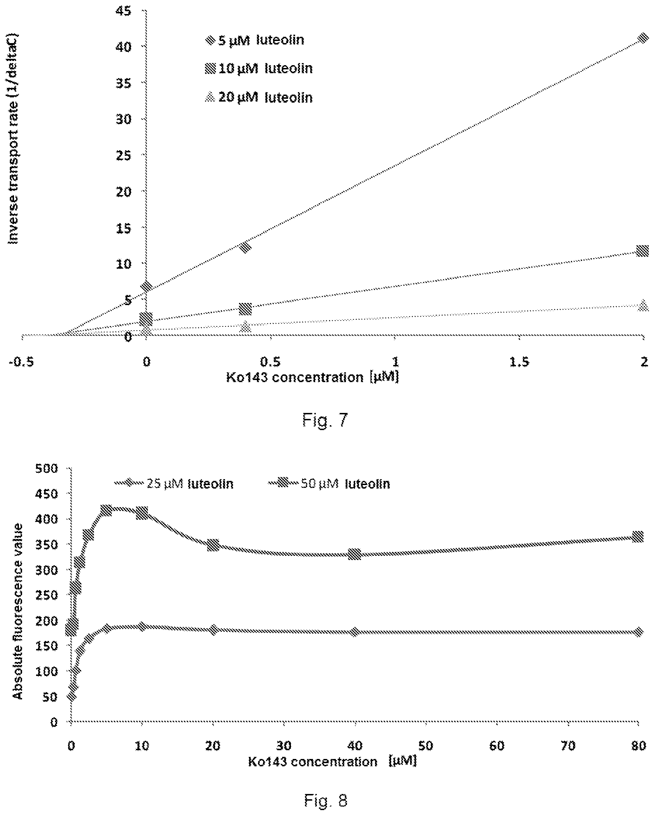

24 hours before the experiment, cells of the SB MDCKII-BCRP cell line (purchased from Solvo Biotechnology) in continuous in vitro culture, exhibiting stable overexpression of human ABCG2 protein, were plated on a standard black 96-well polystyrene microplate with surface modified for cell culture (product from Thermo Scientific Nunc cat. no. 137101) with 50,000 cells per well in standard DMEM culture medium (product from LifeTechnologies cat. no. 32430) supplemented with 10% fetal bovine serum. The experiment was started by removing the culture medium from the cells and replacing it with medium of the same composition (in volume of 100 µl) supplemented with luteolin with a final concentration of 5, 10 or 20 µM, as well as with the Ko143 compound (known inhibitor of the ABCG2 protein, product from Sigma Aldrich, cat. no. K2144) with a final concentration of 0, 0.4, 2 or 10 µM. Medium with each of the concentration combinations for luteolin and Ko143 was added to five wells on the microplate. The cells were then incubated for 1 h in a standard incubator for in vitro cell culture in temperature of 37°C. The incubation was followed by removing the medium and the cells were washed twice with portions of 50 µl of buffered saline (PBS, product from Life Technologies cat. no. 10010) in temperature of 4°C. Then the cells were lysed (solubilized) in 100 µl of PBS with the following additives: 0.5% (v/v) Triton X-100 surfactant (product from Sigma Aldrich, cat. no. T8787) and 0.25 mg/ml 2-aminoethyl-diphenylborinate (APB, CAS no. 208-366-5, product from Sigma Aldrich, cat. no. D9754). The compound APB was added to the PBS solution from a stock solution in DMSO with a concentration of 250 mg/ml permanently stored in temperature of -20°C. The cells were lysed by shaking the microplate in room temperature for 5 minutes. This was followed by measurement of fluorescence (deriving from conjugates of the polyphenolic compounds with APB) using a fluorimetric microplate reader (device EnVision from Perkin Elmer) with excitation wavelength of 485 nm and emission wavelength of 579 nm. Fig. 6 shows the results for the relationshipbetween fluorescence and inhibitor concentration, showing saturation kinetics typical for reversible inhibitors. Accordingly, mathematical transformation of the obtained results was performed in order to get a Dixon plot (dependence of inverse rate of transport on inhibitor concentration for selected substrate concentrations) and to obtain the value of inhibition constant (Ki) therefrom. Since the difference in luteolin concentration outside and inside the cell (deltaC) is proportional to the rate of active transport by ABCG2 at the steady state, and it has been previously shown that in the presence of 10 µM Ko143 there is a complete inhibition of transport and equalization of concentrations, the difference between luteolin fluorescence in the lysate after incubation with 10 µM Ko143 and after incubation with a given concentration (indicated on the graph) was taken as the measure of the transport rate. Fig. 7 shows a Dixon plot, wherein the lines determined by the experimental results crossed inside the II quadrant of the graph (indicating a competitive nature of the inhibition of ABCG2 by Ko143), and the averaged absolute values of the abscissa for the intersection points of the three lines determined by the experimental results were used to calculate the value of inhibition constant (Ki) - 342 nM, which is consistent with literature data.

Fig. 6 demonstrates the dependence of fluorescence of the cell lysate on luteolin concentration as a substrate for the ABCG2 protein and on Ko143 concentration as an inhibitor of that transport. Mean ± the standard error of the mean, n=5.

Fig. 7 demonstrates a Dixon plot for determination of the inhibition constant for the inhibition of luteolin transport by ABCG2 by means of Ko143. Mean ± the standard error of the mean, n=5.

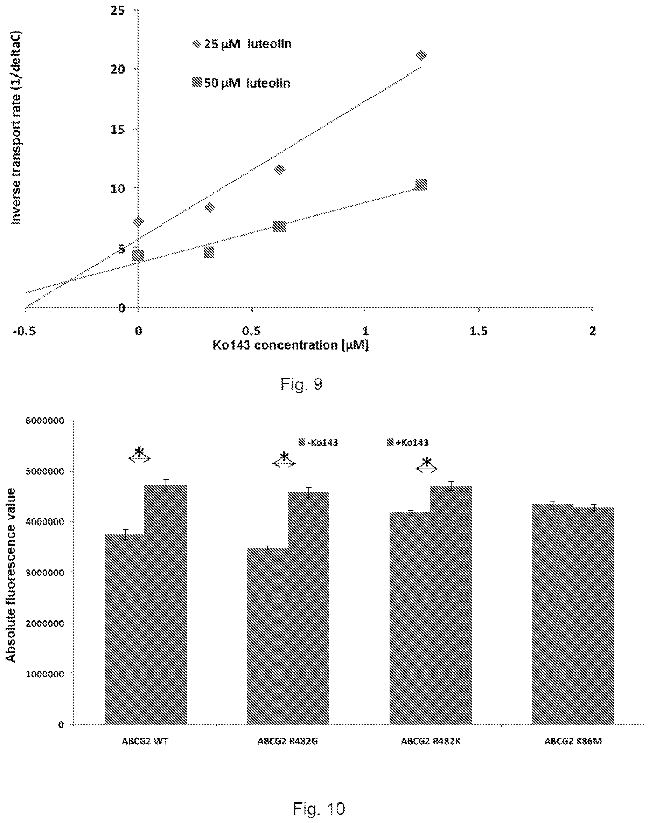

In order to confirm the versatility of the invented method in respect of the fluorescence measurement method, an analogous experiment was conducted using a high content screening platform (HCS), namely an automated fluorescence microscope with specialized software for determination of average fluorescence for the objects in the visual field. For this purpose, 24 hours before the experiment, cells of the SB MDCKII-BCRP cell line (purchased from Solvo Biotechnology), exhibiting stable overexpression of human ABCG2 protein, were plated on a black 96-well polystyrene microplate with a thin (adapted for microscopic observations) glass bottom (product from Greiner cat. no.655 892) with 50,000 cells per well in standard DMEM culture medium (product from LifeTechnologies cat. no. 32430) supplemented with 10% fetal bovine serum. The experiment was started by removing the culture medium from the cells and replacing it with medium of the same composition (in volume of 100 µl) supplemented with luteolin with a final concentration of 25 or 50 µM, as well as with the Ko143 compound (known inhibitor of the ABCG2 protein, product from Sigma Aldrich, cat. no.K2144) with a final concentration of 0.3125 µM to 80 µM (and 0 µM). Medium with each of the concentration combinations for luteolin and Ko143 was added to three wells on the microplate. The cells were then incubated for 1 h in a standard incubator for in vitro cell culture in temperature of 37°C. The incubation was followed by removing the medium and the cells were washed twice with portions of 50 µl of buffered saline (PBS, product from Life Technologies cat. no. 10010) in temperature of 4°C. Then the cells were covered with 100 µl of PBS with the addition of 0.25 mg/ml 2-aminoethyl-diphenylborinate (APB, CAS no. 208-366-5, product from Sigma Aldrich, cat. no. D9754). The compound APB was added to the PBS solution from a stock solution in DMSO with a concentration of 250 mg/ml permanently stored in temperature of -20°C. The microplate was shaken in room temperature for 5 minutes and measured with the ArrayScanVTi device from Thermo FischerCellomics using the incorporated software allowing to identify fluorescent (in the yellow channel) objects (cells) and to determine the average fluorescence for the cells of the whole microplate well. Fig. 8 shows the results for the relationship between fluorescence and inhibitor concentration, confirming the results obtained with a microplate reader. Accordingly, mathematical transformation of the obtained results was performed again in order to get a Dixon plot and to obtain the value of inhibition constant (Ki) therefrom. Again, the difference between luteolin fluorescence in the lysate after incubation with 10 µM Ko143 and after incubation with a given concentration (indicated on the graph) was taken as the measure of the transport rate. Fig. 9 shows a Dixon plot, wherein the lines determined by the experimental results again crossed inside the II quadrant of the graph (indicating a competitive nature of the inhibition of ABCG2 by Ko143), and the averaged absolute values of the abscissa for the intersection points of the three lines determined by the experimental results were used to calculate the value of inhibition constant (Ki) - 313 nM, which is consistent with the result obtained using a microplate reader and literature data.

Fig. 8 demonstrates the dependence of average fluorescence of the cells on luteolin concentration as a substrate for the ABCG2 protein and on Ko143 concentration as an inhibitor of that transport. Mean ± the standard error of the mean, n=3.

Fig. 9 demonstrates a Dixon plot for determination of the inhibition constant for the inhibition of luteolin transport by ABCG2 by means of Ko143. Mean ± the standard error of the mean, n=3.

### Example 4. Testing transport activity of sequential variants of the ABCG2 protein

A plasmid expression vector for the ABCG2 protein was prepared using standard molecular biology techniques. The vector was obtained by cloning of a sequence encoding human ABCG2 and (as an N-terminal fusion protein within the same open reading frame) a sequence encoding TagGFP2 fluorescent protein (obtained from the pTagGFP2-C plasmid produced by Evrogen, cat. no. FP191) into the pcDNA6.2/V5 plasmid. Subsequently, a series of expression vectors for sequential variants of the ABCG2 protein were prepared by performing site-directed mutagenesis in the codons coding particular amino acids in this sequence. For this purpose the Quickchange Lightning kit for site-directed mutagenesis manufactered by AgilentStratagene (cat. no. 210513) was used, in full compliance with the manufacturer's instructions. In this way, the vectors encoding the following sequential variants of the ABCG2 protein were obtained (letters denote the standard IUPAC codes for amino acid residues changed with regard to the wild-type sequence, the number denotes the position of the amino acid residue in the polypeptide chain from the N-terminus of the protein): R482G,R482K, K86M.

96 hours before the experiment, cells of the HeLa cell line (purchased from LGC Standards) in continuous in vitro culture, exhibiting weak expression of human transporters from the ABC superfamily, were plated on a standard 6-well polystyrene microplate with surface modified for cell culture (product from Thermo Scientific Nunc cat. no. 140675) with 900,000 cells per well in standard DMEM culture medium (product from LifeTechnologies cat. no. 32430) supplemented with 10% fetal bovine serum. 72 hours before the experiment, the cells were chemically transfected with the above mentioned plasmids using the FuGene HD reagent (product from Promega, cat. no. E2311). The transfection mixture contained 150 µl of OptiMEM culture medium (product from Life Technologies, cat. no. 31985070), 3.3 µg of plasmid DNA and 9.9 µl of Fugene HD. The mixture was vortexed, incubated for 5 minutes and added to 2 ml of medium and the cells on the 6-well plate. 24 hours before the experiment, the cells were plated on a standard black 96-well polystyrene microplate with surface modified for cell culture (product from Thermo Scientific Nunc cat. no. 137101) with 50,000 cells per well in standard DMEM culture medium (product from LifeTechnologies cat. no. 32430) supplemented with 10% fetal bovine serum. The experiment was started by removing the culture medium from the cells and replacing it with medium of the same composition (in volume of 100 µl) supplemented with luteolin with a final concentration of 10 µM, as well as with the K0143 compound (known inhibitor of the ABCG2 protein, product from Sigma Aldrich, cat. no. K2144) with a final concentration of 0 or 10 µM. Medium with each of the concentration combinations for luteolin and Ko143 was added to five wells on the microplate. The cells were then incubated for 1 h in a standard incubator for in vitro cell culture in temperature of 37°C. The incubation was followed by removing the medium and the cells were washed twice with portions of 50 µl of buffered saline (PBS, product from Life Technologies cat. no. 10010) in temperature of 4°C. Then the cells were lysed (solubilized) in 100 µl of PBS with the following additives: 0.5% (v/v) Triton X-100 surfactant (product from Sigma Aldrich, cat. no. T8787) and 0.25 mg/ml 2-aminoethyl-diphenylborinate (APB, CAS no. 208-366-5, product from Sigma Aldrich, cat. no. D9754). The compound APB was added to the PBS solution from a stock solution in DMSO with a concentration of 250 mg/ml permanently stored in temperature of -20°C. The cells were lysed by shaking the microplate in room temperature for 5 minutes. This was followed by measurement of fluorescence (deriving from conjugates of the polyphenolic compounds with APB) using a fluorimetric microplate reader (device EnVision from Perkin Elmer) with excitation wavelength of 485 nm and emission wavelength of 579 nm. The results are shown on Fig. 10. Statistically significantly higher value of fluorescence in the presence of Ko143, a known inhibitor of substrate transport by the ABCG2 protein, suggests that the particular sequential variant of the ABCG2 protein exhibited a marked transport activity towards luteolin. Based on the obtained results, it may be inferred that the ABCG2 proteins in the following variants: wild-type and R482G show a similar, maximum transport activity. The transport activity towards luteolin for the R482K protein variant is slightly decreased in relation to the wild-type. In contrast, the K86M variant, not having the lysine residue required for the enzymatic activity, did not show any transport activity in the applied test. These results are consistent with those expected based on the literature.

Fig. 10 demonstrates fluorescence of the APB and luteolin conjugate for cell lysates of HeLa cells, exhibiting a temporary overexpression of the variants of the ABCG2 transport protein (mean ± the standard error of the mean, n=5; statistically significant differences at the significance level <0.05 are marked with * symbol).

## Claims

1. In vitro use of 2-aminoethyl-diphenylborinate for studying the activity of the ABCG2 protein.

2. The use according to claim 1, **characterized in that** studying the activity of the ABCG2 protein is used for identifying new ABCG2 substrates among polyphenolic compounds.

3. The use according to claim 1, **characterized in that** studying the activity of the ABCG2 protein is used for characterizing enzymatic or kinetic properties of the studied ABCG2 variants, in particular sequential variants, chemically modified variants, variants from different species.

4. The use according to claim 1, **characterized in that** studying the activity of the ABCG2 protein is used for determining the level of ABCG2 activity in the studied cells, particularly in tissues from biopsies, preferably in cancer cells preparations.

5. The use according to claim 1, **characterized in that** studying the activity of the ABCG2 protein is used for identifying ABCG2 inhibitors, particularly by screening chemical libraries.

6. The use according to claim 1, **characterized in that** studying the activity of the ABCG2 protein is used for detecting pharmacokinetic interactions with ABCG2, particularly ABCG2 inhibition or susceptibility to transport by ABCG2, for compounds of pharmacological importance, such as medicaments, food ingredients, para-pharmaceuticals.

7. The use according to claim 1, **characterized in that** studying the activity of the ABCG2 protein is used for determining the effect of physical factors, preferably temperature, irradiation or osmotic pressure, on ABCG2 activity.

8. An in vitro method of studying the activity of the ABCG2 protein in a studied cell, **characterized in that** the studied cell is incubated with a solution comprising a polyphenolic compound and 2-aminoethyl-diphenylborinate, which is followed by measuring the obtained fluorescence for the studied cell or a lysate thereof, wherein the measured fluorescence level is a measure of the transport activity levels of the ABCG2 protein in the studied cell.

9. The method according to claim 8, **characterized in that** the polyphenolic compound is luteolin.

10. The method according to claim 8, **characterized in that** a cell of a cell line, for which transport activity by the ABCG2 protein was established, is contacted with a solution comprising a polyphenolic compound and 2-aminoethyl-diphenylborinate in the presence of an ABCG2 protein inhibitor, which is followed by measuring the obtained fluorescence for the studied cell, wherein the measured fluorescence level is a measure of the effect of the aforementioned inhibitor on the transport activity levels of the ABCG2 protein in the studied cell, wherein preferably the ABCG2 protein inhibitor is a known inhibitor for this protein, particularly Ko143.

11. An in vitro method of determining susceptibility to transport by ABCG2 for the studied polyphenolic compound, **characterized in that** a cell expressing the ABCG2 protein in incubated with a solution comprising the studied polyphenolic compound and 2-aminoethyl-diphenylborinate, which is followed by measuring the obtained fluorescence for the studied cell or a lysate thereof, wherein the measured fluorescence level is a measure of the susceptibility to transport by ABCG2 for the studied polyphenolic compound.

## Patentansprüche

1. In-vitro-Verwendung von 2-Aminoethyldiphenylborinat zur Untersuchung der Aktivität des ABCG2-Proteins.

2. Verwendung gemäß Anspruch 1, **dadurch gekennzeichnet, dass** die Untersuchung der Aktivität des ABCG2-Proteins verwendet wird, um unter polyphenolischen Verbindungen neue ABCG2-Substrate zu identifizieren.

3. Verwendung gemäß Anspruch 1, **dadurch gekennzeichnet, dass** die Untersuchung der Aktivität des ABCG2-Proteins verwendet wird, um enzymatische oder kinetische Eigenschaften der untersuchten ABCG2-Varianten, insbesondere sequentieller Varianten, chemisch modifizierter Varianten, Varianten von unterschiedlichen Spezies, zu charakterisieren.

4. Verwendung gemäß Anspruch 1, **dadurch gekennzeichnet, dass** die Untersuchung der Aktivität des ABCG2-Proteins verwendet wird, um das Ausmaß der ABCG2-Aktivität in den untersuchten Zellen, insbesondere in Geweben aus Biopsien, vorzugsweise in Präparaten von Krebszellen, zu bestimmen.

5. Verwendung gemäß Anspruch 1, **dadurch gekennzeichnet, dass** die Untersuchung der Aktivität des ABCG2-Proteins verwendet wird, um ABCG2-Inhibitoren zu identifizieren, insbesondere indem man chemische Bibliotheken durchmustert.

6. Verwendung gemäß Anspruch 1, **dadurch gekennzeichnet, dass** die Untersuchung der Aktivität des ABCG2-Proteins verwendet wird, um pharmakokinetische Wechselwirkungen mit ABCG2, insbesondere ABCG2-Hemmung oder Anfälligkeit für Transport durch ABCG2, für Verbindungen mit pharmakologischer Bedeutung, wie Medikamente, Nahrungszutaten, Parapharmaka, nachzuweisen.

7. Verwendung gemäß Anspruch 1, **dadurch gekennzeichnet, dass** die Untersuchung der Aktivität des ABCG2-Proteins verwendet wird, um die Wirkung physikalischer Faktoren, vorzugsweise Temperatur, Bestrahlung oder osmotischer Druck, auf die ABCG2-Aktivität zu bestimmen.

8. In-Vitro-Verfahren zur Untersuchung der Aktivität des ABCG2-Proteins in einer untersuchten Zelle, **dadurch gekennzeichnet, dass** die untersuchte Zelle mit einer Lösung, die eine polyphenolische Verbindung und 2-Aminoethyldiphenylborinat umfasst, inkubiert wird und anschließend die bei der untersuchten Zelle oder einem Lysat davon erhaltene Fluoreszenz gemessen wird, wobei die gemessene Fluoreszenzintensität ein Maß für das Niveau der Transportaktivität des ABCG2-Proteins in der untersuchten Zelle ist.

9. Verfahren gemäß Anspruch 8, **dadurch gekennzeichnet, dass** es sich bei der polyphenolischen Verbindung um Luteolin handelt.

10. Verfahren gemäß Anspruch 8, **dadurch gekennzeichnet, dass** eine Zelle einer Zelllinie, bei der die Transportaktivität des ABCG2-Proteins festgestellt wurde, in Gegenwart eines ABCG2-Protein-Inhibitors mit einer Lösung, die eine polyphenolische Verbindung und 2-Aminoethyldiphenylborinat umfasst, in Kontakt gebracht wird, wobei anschließend die bei der untersuchten Zelle erhaltene Fluoreszenz gemessen wird, wobei die gemessene Fluoreszenzintensität ein Maß für die Wirkung des oben genannten Inhibitors auf das Niveau der Transportaktivität des ABCG2-Proteins in der untersuchten Zelle ist, wobei es sich vorzugsweise bei dem ABCG2-Protein-Inhibitor um einen bekannten Inhibitor für dieses Protein, insbesondere Ko143, handelt.

11. In-vitro-Verfahren zur Bestimmung der Anfälligkeit der untersuchten polyphenolischen Verbindung für Transport durch ABCG2, **dadurch gekennzeichnet, dass** eine Zelle, die das ABCG2-Protein exprimiert, mit einer Lösung, die die untersuchte polyphenolische Verbindung und 2-Aminoethyldiphenylborinat umfasst, inkubiert wird und anschließend die bei der untersuchten Zelle oder einem Lysat davon erhaltene Fluoreszenz gemessen wird, wobei die gemessene Fluoreszenzintensität ein Maß für die Anfälligkeit der untersuchten polyphenolischen Verbindung für Transport durch ABCG2 ist.

## Revendications

1. Utilisation *in vitro* de diphénylborinate de 2-aminoéthyle pour étudier l'activité de la protéine ABCG2.

2. Utilisation selon la revendication 1, **caractérisée en ce que** l'étude de l'activité de la protéine ABCG2 est utilisée pour identifier de nouveaux substrats de l'ABCG2 parmi des composés polyphénoliques.

3. Utilisation selon la revendication 1, **caractérisée en ce que** l'étude de l'activité de la protéine ABCG2 est utilisée pour caractériser les propriétés enzymatiques ou cinétiques des variants étudiés de l'ABCG2, en particulier des variants séquentiels, des variants modifiés chimiquement, des variants provenant d'espèces différentes.

4. Utilisation selon la revendication 1, **caractérisée en ce que** l'étude de l'activité de la protéine ABCG2 est utilisée pour déterminer le taux d'activité de l'ABCG2 dans les cellules étudiées, particulièrement dans des tissus provenant de biopsies, de préférence dans des préparations de cellules cancéreuses.

5. Utilisation selon la revendication 1, **caractérisée en ce que** l'étude de l'activité de la protéine ABCG2 est utilisée pour identifier des inhibiteurs de l'ABCG2, particulièrement par criblage de banques de produits chimiques.

6. Utilisation selon la revendication 1, **caractérisée en ce que** l'étude de l'activité de la protéine ABCG2 est utilisée pour détecter les interactions pharmacocinétiques avec l'ABCG2, particulièrement l'inhibition de l'ABCG2 ou la susceptibilité au transport par l'ABCG2, pour des composés d'importance pharmacologique, tels que des médicaments, des ingrédients alimentaires, des produits parapharmaceutiques.

7. Utilisation selon la revendication 1, **caractérisée en ce que** l'étude de l'activité de la protéine ABCG2 est utilisée pour déterminer l'effet de facteurs physiques, de préférence la température, l'irradiation ou la pression osmotique, sur l'activité de l'ABCG2.

8. Procédé *in vitro* d'étude de l'activité de la protéine ABCG2 dans une cellule étudiée, **caractérisé en ce que** la cellule étudiée est incubée avec une solution comprenant un composé polyphénolique et du diphényl-borinate de 2-aminoéthyle, ceci suivi de la mesure de la fluorescence obtenue pour la cellule étudiée ou son lysat, dans lequel le taux de fluorescence mesuré est une mesure des taux d'activité de transport de la protéine ABCG2 dans la cellule étudiée.

9. Procédé selon la revendication 8, **caractérisé en ce que** le composé polyphénolique est la lutéoline.

10. Procédé selon la revendication 8, **caractérisé en ce qu'**une cellule d'une lignée cellulaire, pour laquelle l'activité de transport par la protéine ABCG2 a été établie, est mise en contact avec une solution comprenant un composé polyphénolique et du diphényl-borinate de 2-aminoéthyle en présence d'un inhibiteur de la protéine ABCG2, ceci suivi de la mesure de la fluorescence obtenue pour la cellule étudiée, dans lequel le taux de fluorescence mesuré est une mesure de l'effet de l'inhibiteur mentionné ci-avant sur les taux d'activité de transport de la protéine ABCG2 dans la cellule étudiée, dans lequel de préférence l'inhibiteur de la protéine ABCG2 est un inhibiteur connu pour cette protéine, particulièrement Ko143.

11. Procédé *in vitro* de détermination de la susceptibilité au transport par l'ABCG2 pour le composé polyphénolique étudié, **caractérisé en ce qu'**une cellule exprimant la protéine ABCG2 est incubée avec une solution comprenant le composé polyphénolique étudié et du diphénylborinate de 2-aminoéthyle, ceci suivi de la mesure de la fluorescence obtenue pour la cellule étudiée ou son lysat, dans lequel le taux de fluorescence mesuré est une mesure de la susceptibilité au transport par l'ABCG2 pour le composé polyphénolique étudié.


### SEQUENCE LISTING

<110> Uniwersytet £ódzki
<120> Method of studying ABCG2 protein activity and use of 2-aminoethyl diphenylborinate
<130> PZ/2430/RW/PCT
<160> 4
<170> PatentIn version 3.5
<210> 1
   <211> 655
   <212> PRT
   <213> artificial
<220>
   <223> ABCG2WT
<400> 1
   Met Ser Ser Ser Asn Val Glu Val Phe Ile Pro Val Ser Gln Gly Asn
   1               5                   10                  15
   Thr Asn Gly Phe Pro Ala Thr Ala Ser Asn Asp Leu Lys Ala Phe Thr
               20                  25                  30
   Glu Gly Ala Val Leu Ser Phe His Asn Ile Cys Tyr Arg Val Lys Leu
           35                  40                  45
   Lys Ser Gly Phe Leu Pro Cys Arg Lys Pro Val Glu Lys Glu Ile Leu
       50                  55                  60
   Ser Asn Ile Asn Gly Ile Met Lys Pro Gly Leu Asn Ala Ile Leu Gly
   65                  70                  75                  80
   Pro Thr Gly Gly Gly Lys Ser Ser Leu Leu Asp Val Leu Ala Ala Arg
                   85                  90                  95
   Lys Asp Pro Ser Gly Leu Ser Gly Asp Val Leu Ile Asn Gly Ala Pro
               100                 105                 110
   Arg Pro Ala Asn Phe Lys Cys Asn Ser Gly Tyr Val Val Gln Asp Asp
           115                 120                 125
   Val Val Met Gly Thr Leu Thr Val Arg Glu Asn Leu Gln Phe Ser Ala
       130                 135                 140
   Ala Leu Arg Leu Ala Thr Thr Met Thr Asn His Glu Lys Asn Glu Arg
   145                 150                 155                 160
   Ile Asn Arg Val Ile Gln Glu Leu Gly Leu Asp Lys Val Ala Asp Ser
                   165                 170                 175
   Lys Val Gly Thr Gln Phe Ile Arg Gly Val Ser Gly Gly Glu Arg Lys
               180                 185                 190
   Arg Thr Ser Ile Gly Met Glu Leu Ile Thr Asp Pro Ser Ile Leu Phe
           195                 200                 205
   Leu Asp Glu Pro Thr Thr Gly Leu Asp Ser Ser Thr Ala Asn Ala Val
       210                 215                 220
   Leu Leu Leu Leu Lys Arg Met Ser Lys Gln Gly Arg Thr Ile Ile Phe
   225                 230                 235                 240
   Ser Ile His Gln Pro Arg Tyr Ser Ile Phe Lys Leu Phe Asp Ser Leu
                   245                 250                 255
   Thr Leu Leu Ala Ser Gly Arg Leu Met Phe His Gly Pro Ala Gln Glu
               260                 265                 270
   Ala Leu Gly Tyr Phe Glu Ser Ala Gly Tyr His Cys Glu Ala Tyr Asn
           275                 280                 285
   Asn Pro Ala Asp Phe Phe Leu Asp Ile Ile Asn Gly Asp Ser Thr Ala
       290                 295                 300
   Val Ala Leu Asn Arg Glu Glu Asp Phe Lys Ala Thr Glu Ile Ile Glu
   305                 310                 315                 320
   Pro Ser Lys Gln Asp Lys Pro Leu Ile Glu Lys Leu Ala Glu Ile Tyr
                   325                 330                 335
   Val Asn Ser Ser Phe Tyr Lys Glu Thr Lys Ala Glu Leu His Gln Leu
               340                 345                 350
   Ser Gly Gly Glu Lys Lys Lys Lys Ile Thr Val Phe Lys Glu Ile Ser
           355                 360                 365
   Tyr Thr Thr Ser Phe Cys His Gln Leu Arg Trp Val Ser Lys Arg Ser
       370                 375                 380
   Phe Lys Asn Leu Leu Gly Asn Pro Gln Ala Ser Ile Ala Gln Ile Ile
   385                 390                 395                 400
   Val Thr Val Val Leu Gly Leu Val Ile Gly Ala Ile Tyr Phe Gly Leu
                   405                 410                 415
   Lys Asn Asp Ser Thr Gly Ile Gln Asn Arg Ala Gly Val Leu Phe Phe
               420                 425                 430
   Leu Thr Thr Asn Gln Cys Phe Ser Ser Val Ser Ala Val Glu Leu Phe
           435                 440                 445
   Val Val Glu Lys Lys Leu Phe Ile His Glu Tyr Ile Ser Gly Tyr Tyr
       450                 455                 460
   Arg Val Ser Ser Tyr Phe Leu Gly Lys Leu Leu Ser Asp Leu Leu Pro
   465                 470                 475                 480
   Met Arg Met Leu Pro Ser Ile Ile Phe Thr Cys Ile Val Tyr Phe Met
                   485                 490                 495
   Leu Gly Leu Lys Pro Lys Ala Asp Ala Phe Phe Val Met Met Phe Thr
               500                 505                 510
   Leu Met Met Val Ala Tyr Ser Ala Ser Ser Met Ala Leu Ala Ile Ala
           515                 520                 525
   Ala Gly Gln Ser Val Val Ser Val Ala Thr Leu Leu Met Thr Ile Cys
       530                 535                 540
   Phe Val Phe Met Met Ile Phe Ser Gly Leu Leu Val Asn Leu Thr Thr
   545                 550                 555                 560
   Ile Ala Ser Trp Leu Ser Trp Leu Gln Tyr Phe Ser Ile Pro Arg Tyr
                   565                 570                 575
   Gly Phe Thr Ala Leu Gln His Asn Glu Phe Leu Gly Gln Asn Phe Cys
               580                 585                 590
   Pro Gly Leu Asn Ala Thr Gly Asn Asn Pro Cys Asn Tyr Ala Thr Cys
           595                 600                 605
   Thr Gly Glu Glu Tyr Leu Val Lys Gln Gly Ile Asp Leu Ser Pro Trp
       610                 615                 620
   Gly Leu Trp Lys Asn His Val Ala Leu Ala Cys Met Ile Val Ile Phe
   625                 630                 635                 640
   Leu Thr Ile Ala Tyr Leu Lys Leu Leu Phe Leu Lys Lys Tyr Ser
                   645                 650                 655
<210> 2
   <211> 655
   <212> PRT
   <213> artificial
<220>
   <223> ABCG2 R482G
<400> 2
   Met Ser Ser Ser Asn Val Glu Val Phe Ile Pro Val Ser Gln Gly Asn
   1               5                   10                  15
   Thr Asn Gly Phe Pro Ala Thr Ala Ser Asn Asp Leu Lys Ala Phe Thr
               20                  25                  30
   Glu Gly Ala Val Leu Ser Phe His Asn Ile Cys Tyr Arg Val Lys Leu
           35                  40                  45
   Lys Ser Gly Phe Leu Pro Cys Arg Lys Pro Val Glu Lys Glu Ile Leu
       50                  55                  60
   Ser Asn Ile Asn Gly Ile Met Lys Pro Gly Leu Asn Ala Ile Leu Gly
   65                  70                  75                  80
   Pro Thr Gly Gly Gly Lys Ser Ser Leu Leu Asp Val Leu Ala Ala Arg
                   85                  90                  95
   Lys Asp Pro Ser Gly Leu Ser Gly Asp Val Leu Ile Asn Gly Ala Pro
               100                 105                 110
   Arg Pro Ala Asn Phe Lys Cys Asn Ser Gly Tyr Val Val Gln Asp Asp
           115                 120                 125
   Val Val Met Gly Thr Leu Thr Val Arg Glu Asn Leu Gln Phe Ser Ala
       130                 135                 140
   Ala Leu Arg Leu Ala Thr Thr Met Thr Asn His Glu Lys Asn Glu Arg
   145                 150                 155                 160
   Ile Asn Arg Val Ile Gln Glu Leu Gly Leu Asp Lys Val Ala Asp Ser
                   165                 170                 175
   Lys Val Gly Thr Gln Phe Ile Arg Gly Val Ser Gly Gly Glu Arg Lys
               180                 185                 190
   Arg Thr Ser Ile Gly Met Glu Leu Ile Thr Asp Pro Ser Ile Leu Phe
           195                 200                 205
   Leu Asp Glu Pro Thr Thr Gly Leu Asp Ser Ser Thr Ala Asn Ala Val
       210                 215                 220
   Leu Leu Leu Leu Lys Arg Met Ser Lys Gln Gly Arg Thr Ile Ile Phe
   225                 230                 235                 240
   Ser Ile His Gln Pro Arg Tyr Ser Ile Phe Lys Leu Phe Asp Ser Leu
                   245                 250                 255
   Thr Leu Leu Ala Ser Gly Arg Leu Met Phe His Gly Pro Ala Gln Glu
               260                 265                 270
   Ala Leu Gly Tyr Phe Glu Ser Ala Gly Tyr His Cys Glu Ala Tyr Asn
           275                 280                 285
   Asn Pro Ala Asp Phe Phe Leu Asp Ile Ile Asn Gly Asp Ser Thr Ala
       290                 295                 300
   Val Ala Leu Asn Arg Glu Glu Asp Phe Lys Ala Thr Glu Ile Ile Glu
   305                 310                 315                 320
   Pro Ser Lys Gln Asp Lys Pro Leu Ile Glu Lys Leu Ala Glu Ile Tyr
                   325                 330                 335
   Val Asn Ser Ser Phe Tyr Lys Glu Thr Lys Ala Glu Leu His Gln Leu
               340                 345                 350
   Ser Gly Gly Glu Lys Lys Lys Lys Ile Thr Val Phe Lys Glu Ile Ser
           355                 360                 365
   Tyr Thr Thr Ser Phe Cys His Gln Leu Arg Trp Val Ser Lys Arg Ser
       370                 375                 380
   Phe Lys Asn Leu Leu Gly Asn Pro Gln Ala Ser Ile Ala Gln Ile Ile
   385                 390                 395                 400
   Val Thr Val Val Leu Gly Leu Val Ile Gly Ala Ile Tyr Phe Gly Leu
                   405                 410                 415
   Lys Asn Asp Ser Thr Gly Ile Gln Asn Arg Ala Gly Val Leu Phe Phe
               420                 425                 430
   Leu Thr Thr Asn Gln Cys Phe Ser Ser Val Ser Ala Val Glu Leu Phe
           435                 440                 445
   Val Val Glu Lys Lys Leu Phe Ile His Glu Tyr Ile Ser Gly Tyr Tyr
       450                 455                 460
   Arg Val Ser Ser Tyr Phe Leu Gly Lys Leu Leu Ser Asp Leu Leu Pro
   465                 470                 475                 480
   Met Gly Met Leu Pro Ser Ile Ile Phe Thr Cys Ile Val Tyr Phe Met
   485 490 495
   Leu Gly Leu Lys Pro Lys Ala Asp Ala Phe Phe Val Met Met Phe Thr
   500 505 510
   Leu Met Met Val Ala Tyr Ser Ala Ser Ser Met Ala Leu Ala Ile Ala
   515 520 525
   Ala Gly Gln Ser Val Val Ser Val Ala Thr Leu Leu Met Thr Ile Cys
   530 535 540
   Phe Val Phe Met Met Ile Phe Ser Gly Leu Leu Val Asn Leu Thr Thr
   545 550 555 560
   Ile Ala Ser Trp Leu Ser Trp Leu Gln Tyr Phe Ser Ile Pro Arg Tyr
   565 570 575
   Gly Phe Thr Ala Leu Gln His Asn Glu Phe Leu Gly Gln Asn Phe Cys
   580 585 590
   Pro Gly Leu Asn Ala Thr Gly Asn Asn Pro Cys Asn Tyr Ala Thr Cys
   595 600 605
   Thr Gly Glu Glu Tyr Leu Val Lys Gln Gly Ile Asp Leu Ser Pro Trp
   610 615 620
   Gly Leu Trp Lys Asn His Val Ala Leu Ala Cys Met Ile Val Ile Phe
   625 630 635 640
   Leu Thr Ile Ala Tyr Leu Lys Leu Leu Phe Leu Lys Lys Tyr Ser
   645 650 655
<210> 3
   <211> 655
   <212> PRT
   <213> artificial
<220>
   <223> ABCG2 R482K
<400> 3
   Met Ser Ser Ser Asn Val Glu Val Phe Ile Pro Val Ser Gln Gly Asn
   1 5 10 15
   Thr Asn Gly Phe Pro Ala Thr Ala Ser Asn Asp Leu Lys Ala Phe Thr
   20 25 30
   Glu Gly Ala Val Leu Ser Phe His Asn Ile Cys Tyr Arg Val Lys Leu
           35                  40                  45
   Lys Ser Gly Phe Leu Pro Cys Arg Lys Pro Val Glu Lys Glu Ile Leu
       50                  55                  60
   Ser Asn Ile Asn Gly Ile Met Lys Pro Gly Leu Asn Ala Ile Leu Gly
   65                  70                  75                  80
   Pro Thr Gly Gly Gly Lys Ser Ser Leu Leu Asp Val Leu Ala Ala Arg
                   85                  90                  95
   Lys Asp Pro Ser Gly Leu Ser Gly Asp Val Leu Ile Asn Gly Ala Pro
               100                 105                 110
   Arg Pro Ala Asn Phe Lys Cys Asn Ser Gly Tyr Val Val Gln Asp Asp
           115                 120                 125
   Val Val Met Gly Thr Leu Thr Val Arg Glu Asn Leu Gln Phe Ser Ala
       130                 135                 140
   Ala Leu Arg Leu Ala Thr Thr Met Thr Asn His Glu Lys Asn Glu Arg
   145                 150                 155                 160
   Ile Asn Arg Val Ile Gln Glu Leu Gly Leu Asp Lys Val Ala Asp Ser
                   165                 170                 175
   Lys Val Gly Thr Gln Phe Ile Arg Gly Val Ser Gly Gly Glu Arg Lys
               180                 185                 190
   Arg Thr Ser Ile Gly Met Glu Leu Ile Thr Asp Pro Ser Ile Leu Phe
           195                 200                 205
   Leu Asp Glu Pro Thr Thr Gly Leu Asp Ser Ser Thr Ala Asn Ala Val
       210                 215                 220
   Leu Leu Leu Leu Lys Arg Met Ser Lys Gln Gly Arg Thr Ile Ile Phe
   225                 230                 235                 240
   Ser Ile His Gln Pro Arg Tyr Ser Ile Phe Lys Leu Phe Asp Ser Leu
                   245                 250                 255
   Thr Leu Leu Ala Ser Gly Arg Leu Met Phe His Gly Pro Ala Gln Glu
               260                 265                 270
   Ala Leu Gly Tyr Phe Glu Ser Ala Gly Tyr His Cys Glu Ala Tyr Asn
           275                 280                 285
   Asn Pro Ala Asp Phe Phe Leu Asp Ile Ile Asn Gly Asp Ser Thr Ala
   290 295 300
   Val Ala Leu Asn Arg Glu Glu Asp Phe Lys Ala Thr Glu Ile Ile Glu
   305 310 315 320
   Pro Ser Lys Gln Asp Lys Pro Leu Ile Glu Lys Leu Ala Glu Ile Tyr
   325 330 335
   Val Asn Ser Ser Phe Tyr Lys Glu Thr Lys Ala Glu Leu His Gln Leu
   340 345 350
   Ser Gly Gly Glu Lys Lys Lys Lys Ile Thr Val Phe Lys Glu Ile Ser
   355 360 365
   Tyr Thr Thr Ser Phe Cys His Gln Leu Arg Trp Val Ser Lys Arg Ser
   370 375 380
   Phe Lys Asn Leu Leu Gly Asn Pro Gln Ala Ser Ile Ala Gln Ile Ile
   385 390 395 400
   Val Thr Val Val Leu Gly Leu Val Ile Gly Ala Ile Tyr Phe Gly Leu
   405 410 415
   Lys Asn Asp Ser Thr Gly Ile Gln Asn Arg Ala Gly Val Leu Phe Phe
   420 425 430
   Leu Thr Thr Asn Gln Cys Phe Ser Ser Val Ser Ala Val Glu Leu Phe
   435 440 445
   Val Val Glu Lys Lys Leu Phe Ile His Glu Tyr Ile Ser Gly Tyr Tyr
   450 455 460
   Arg Val Ser Ser Tyr Phe Leu Gly Lys Leu Leu Ser Asp Leu Leu Pro
   465 470 475 480
   Met Lys Met Leu Pro Ser Ile Ile Phe Thr Cys Ile Val Tyr Phe Met
   485 490 495
   Leu Gly Leu Lys Pro Lys Ala Asp Ala Phe Phe Val Met Met Phe Thr
   500 505 510
   Leu Met Met Val Ala Tyr Ser Ala Ser Ser Met Ala Leu Ala Ile Ala
   515 520 525
   Ala Gly Gln Ser Val Val Ser Val Ala Thr Leu Leu Met Thr Ile Cys
           530                 535                 540
   Phe Val Phe Met Met Ile Phe Ser Gly Leu Leu Val Asn Leu Thr Thr
   545                 550                 555                 560
   Ile Ala Ser Trp Leu Ser Trp Leu Gln Tyr Phe Ser Ile Pro Arg Tyr
                   565                 570                 575
   Gly Phe Thr Ala Leu Gln His Asn Glu Phe Leu Gly Gln Asn Phe Cys
               580                 585                 590
   Pro Gly Leu Asn Ala Thr Gly Asn Asn Pro Cys Asn Tyr Ala Thr Cys
           595                 600                 605
   Thr Gly Glu Glu Tyr Leu Val Lys Gln Gly Ile Asp Leu Ser Pro Trp
       610                 615                 620
   Gly Leu Trp Lys Asn His Val Ala Leu Ala Cys Met Ile Val Ile Phe
   625                 630                 635                 640
   Leu Thr Ile Ala Tyr Leu Lys Leu Leu Phe Leu Lys Lys Tyr Ser
                   645                 650                 655
<210> 4
   <211> 655
   <212> PRT
   <213> artificial
<220>
   <223> ABCG2 K86M
<400> 4
   Met Ser Ser Ser Asn Val Glu Val Phe Ile Pro Val Ser Gln Gly Asn
   1               5                   10                  15
   Thr Asn Gly Phe Pro Ala Thr Ala Ser Asn Asp Leu Lys Ala Phe Thr
               20                  25                  30
   Glu Gly Ala Val Leu Ser Phe His Asn Ile Cys Tyr Arg Val Lys Leu
           35                  40                  45
   Lys Ser Gly Phe Leu Pro Cys Arg Lys Pro Val Glu Lys Glu Ile Leu
       50                  55                  60
   Ser Asn Ile Asn Gly Ile Met Lys Pro Gly Leu Asn Ala Ile Leu Gly
   65                  70                  75                  80
   Pro Thr Gly Gly Gly Met Ser Ser Leu Leu Asp Val Leu Ala Ala Arg
                   85                  90                  95
   Lys Asp Pro Ser Gly Leu Ser Gly Asp Val Leu Ile Asn Gly Ala Pro
               100                 105                 110
   Arg Pro Ala Asn Phe Lys Cys Asn Ser Gly Tyr Val Val Gln Asp Asp
           115                 120                 125
   Val Val Met Gly Thr Leu Thr Val Arg Glu Asn Leu Gln Phe Ser Ala
       130                 135                 140
   Ala Leu Arg Leu Ala Thr Thr Met Thr Asn His Glu Lys Asn Glu Arg
   145                 150                 155                 160
   Ile Asn Arg Val Ile Gln Glu Leu Gly Leu Asp Lys Val Ala Asp Ser
                   165                 170                 175
   Lys Val Gly Thr Gln Phe Ile Arg Gly Val Ser Gly Gly Glu Arg Lys
               180                 185                 190
   Arg Thr Ser Ile Gly Met Glu Leu Ile Thr Asp Pro Ser Ile Leu Phe
           195                 200                 205
   Leu Asp Glu Pro Thr Thr Gly Leu Asp Ser Ser Thr Ala Asn Ala Val
       210                 215                 220
   Leu Leu Leu Leu Lys Arg Met Ser Lys Gln Gly Arg Thr Ile Ile Phe
   225                 230                 235                 240
   Ser Ile His Gln Pro Arg Tyr Ser Ile Phe Lys Leu Phe Asp Ser Leu
                   245                 250                 255
   Thr Leu Leu Ala Ser Gly Arg Leu Met Phe His Gly Pro Ala Gln Glu
               260                 265                 270
   Ala Leu Gly Tyr Phe Glu Ser Ala Gly Tyr His Cys Glu Ala Tyr Asn
           275                 280                 285
   Asn Pro Ala Asp Phe Phe Leu Asp Ile Ile Asn Gly Asp Ser Thr Ala
       290                 295                 300
   Val Ala Leu Asn Arg Glu Glu Asp Phe Lys Ala Thr Glu Ile Ile Glu
   305                 310                 315                 320
   Pro Ser Lys Gln Asp Lys Pro Leu Ile Glu Lys Leu Ala Glu Ile Tyr
                   325                 330                 335
   Val Asn Ser Ser Phe Tyr Lys Glu Thr Lys Ala Glu Leu His Gln Leu
   340 345 350
   Ser Gly Gly Glu Lys Lys Lys Lys Ile Thr Val Phe Lys Glu Ile Ser
   355 360 365
   Tyr Thr Thr Ser Phe Cys His Gln Leu Arg Trp Val Ser Lys Arg Ser
   370 375 380
   Phe Lys Asn Leu Leu Gly Asn Pro Gln Ala Ser Ile Ala Gln Ile Ile
   385 390 395 400
   Val Thr Val Val Leu Gly Leu Val Ile Gly Ala Ile Tyr Phe Gly Leu
   405 410 415
   Lys Asn Asp Ser Thr Gly Ile Gln Asn Arg Ala Gly Val Leu Phe Phe
   420 425 430
   Leu Thr Thr Asn Gln Cys Phe Ser Ser Val Ser Ala Val Glu Leu Phe
   435 440 445
   Val Val Glu Lys Lys Leu Phe Ile His Glu Tyr Ile Ser Gly Tyr Tyr
   450 455 460
   Arg Val Ser Ser Tyr Phe Leu Gly Lys Leu Leu Ser Asp Leu Leu Pro
   465 470 475 480
   Met Arg Met Leu Pro Ser Ile Ile Phe Thr Cys Ile Val Tyr Phe Met
   485 490 495
   Leu Gly Leu Lys Pro Lys Ala Asp Ala Phe Phe Val Met Met Phe Thr
   500 505 510
   Leu Met Met Val Ala Tyr Ser Ala Ser Ser Met Ala Leu Ala Ile Ala
   515 520 525
   Ala Gly Gln Ser Val Val Ser Val Ala Thr Leu Leu Met Thr Ile Cys
   530 535 540
   Phe Val Phe Met Met Ile Phe Ser Gly Leu Leu Val Asn Leu Thr Thr
   545 550 555 560
   Ile Ala Ser Trp Leu Ser Trp Leu Gln Tyr Phe Ser Ile Pro Arg Tyr
   565 570 575
   Gly Phe Thr Ala Leu Gln His Asn Glu Phe Leu Gly Gln Asn Phe Cys
   580 585 590
   Pro Gly Leu Asn Ala Thr Gly Asn Asn Pro Cys Asn Tyr Ala Thr Cys
           595                 600                 605
   Thr Gly Glu Glu Tyr Leu Val Lys Gln Gly Ile Asp Leu Ser Pro Trp
       610                 615                 620
   Gly Leu Trp Lys Asn His Val Ala Leu Ala Cys Met Ile Val Ile Phe
   625                 630                 635                 640
   Leu Thr Ile Ala Tyr Leu Lys Leu Leu Phe Leu Lys Lys Tyr Ser
                   645                 650                 655